# EUROPEAN PATENT SPECIFICATION

(11) **EP 0 326 100 B1**
(45) Date of publication and mention of the grant of the patent: **11.09.1996**
(21) Application number: 89101263.5
(22) Date of filing: 25.01.1989
(51) Int. Cl.: G01N 33/537, G01N 33/543, G01N 33/538

(54) **Ion-capture assays and devices**
Ionenfang-Teste und -Vorrichtungen
Essais et dispositifs à capture d'ions

(30) Priority: 29.01.1988 US 150278
(43) Date of publication of application: 02.08.1989
(73) Proprietor: ABBOTT LABORATORIES, Abbott Park, Illinois 60064-3500 (US)
(72) Inventor: Hiltibran, Robert G., Bristol, WI 53104 (US); Jou, Yi-Her, Vernon Hills, IL 60061 (US); Kline, Steven J., Grayslake, IL 60030 (US); Schultz, Steven George, Winthrop Harbor, IL 60096 (US); Stroupe, Stephen Denham, Libertyville, IL 60048 (US)
(74) Representative: Modiano, Guido, Dr.-Ing.

(56) References cited:
- EP-A- 0 077 671
- EP-A- 0 214 909
- EP-A- 0 230 768
- FR-A- 2 387 452
- US-A- 4 935 147
- US-A- 4 948 726

## Description

### BACKGROUND OF THE INVENTION

### Technical Field

This invention relates generally to the field of immunoassay devices and methods. More particularly. the present invention relates to novel methods and products useful in the performance of homogeneous immunoassays.

### Background

Various analytical procedures and devices are commonly employed in assays to determine the presence and/or concentration of substances of interest or clinical significance which may be present in biological fluids or other materials. Such substances are commonly termed "analytes" and can include antibodies, antigens, drugs. hormones, etc.

Immunoassay techniques take advantage of the mechanisms of the immune systems of higher organisms, wherein antibodies are produced in response to the presence of antigens which are pathogenic or foreign to the organisms. One or more antibodies are produced in response to and are capable of reacting with a particular antigen, thereby creating a highly specific reaction mechanism which can be used *in vitro* to determine the presence or concentration of that particular antigen in a biological sample.

The solid phase immunoassay is a commonly used immunoassay technique. There are a number of solid phase immunoassay devices and procedures wherein the presence of an analyte is indicated by means of a reaction on a "solid phase" such as a dipstick, test strip, paper, fiber matrix, 1/4 inch (0.64 cm) bead, or other solid material.

One conventional solid phase enzyme immunoassay configuration uses an anti-analyte antibody (capture antibody) bound to an insoluble solid phase material, such as polystyrene beads or latex microparticles associated with a fibrous matrix. A second anti-analyte antibody is labeled with an enzyme to form a soluble indicator reagent, and the enzyme will react with an enzyme substrate to form a detectable product (the second antibody can-be labeled with a radioisotope, fluorophore, chemilumiphore, or any readily detected signal generator.) The two antibody conjugates form an insoluble ternary immunocomplex ("sandwich") with the analyte when the latter is present in the test sample. Prior to the addition of enzyme substrate and the detection or measurement of product, the immunocomplex is separated from excess indicator reagent and other interfering substances by physically removing the solid phase-bound immunocomplex from the reaction mixture. When the enzyme substrate is added. it reacts with the enzyme portion of the indicator, and the amount of labeled antibody associated with the solid phase bound sandwich is directly proportional to the amount of analyte in the test sample.

An alternative methodology is the competitive assay. The capture mechanism again uses an anti-analyte antibody conjugated to the insoluble solid phase, but enzyme-labeled analyte (rather than a second antibody) is used as an indicator and is simultaneously incubated with the test sample and solid phase. Therefore, in the competitive assay the indicator competes with analyte present in the sample to bind with the solid phase, and binary immunocomplexes are formed, i.e., a solid phase/analyte conjugate and a solid phase/indicator conjugate. In the competitive assay, the quantity of captured indicator reagent is inversely proportional to the amount of analyte present in the sample.

Despite their great utility, there are several disadvantages with such assay methods. First, the heterogenous reaction mixture of soluble and insoluble reagents and fluid test sample can retard the kinetics of the reaction. Lengthy incubation periods may be required for equilibrium to be reached in the reaction mixture between the insoluble solid phase system, the free analyte in the test sample, the soluble indicator reagent, and the newly formed insoluble complex. Second, conventional methods of attaching capture reagents to solid phase materials, such as adsorption, can produce a solid phase which will readily bind substances other than the analyte. This is referred to as nonspecific binding which can interfere with the detection of a positive result. Third, while certain methods such as fluorescence polarization immunoassay (FPIA) and enzyme multiplied immunoassay technique (EMIT) have the advantages of homogeneous assays and overcome some of the problems of solid phase competitive binding immunoassays, they also have the disadvantage of detecting the signal in the presence of sample background.

Relevant as background art to the present invention is EP-A-0 230 768 which discloses assays involving the separation of a substance from a liquid medium by the use of a magnetic field gradient. The assay depends on the coagglutination of magnetic particles and non-magnetic particles to which a member of a specific binding pair is bound, e.g. by covalent binding, as capture reagent. Coagglutination may occur because of ionic interaction between the oppositely charged magnetic and non-magnetic particles.

### SUMMARY OF THE INVENTION

This invention presents novel assay and separation procedures which enables both the capture reagent and the indicator reagent to be in solution to avoid problems with slowed reaction kinetics.

The separation procedure involves a soluble specific binding substance, i.e., capture reagent, that is conjugated to a charged substance and an insoluble solid phase material that is oppositely charged. A fluid sample suspected of containing the analyte is mixed with the capture reagent in solution to form a charged complex. When binding is complete, the solution is contacted to the oppositely charged solid phase material to attract, attach, and separate the newly formed complex from the fluid sample.

Furthermore, the invention can be used for detecting an analyte in a fluid sample, through the addition of an indicator reagent, i.e., a second analyte-specific binding substance which is conjugated to a label capable of producing a detectable signal. The indicator reagent can be used in a competitive assay or in a sandwich assay where it is contacted to the analyte and the capture reagent to form a capture reagent/analyte/indicator reagent complex that is both labeled and charged. The complex then can be separated from the solution by contact with the oppositely charged solid phase material, and the presence or amount of analyte is monitored by detecting the label of the indicator reagent.

In addition, a number of charged solid phase materials can be used as well as specific charged species, especially polymeric ionic substances, which can be coupled to the solid phase material for attracting and attaching to the oppositely charged capture reagent and assay complex. The use of the charged substances produces assay procedures having a highly specific separation method, minimal nonspecific binding, and high sensitivity.

In a known assay technique the capture reagent, typically an antibody, is conjugated to a solid phase, and the fluid test sample is passed through or over the solid phase to allow the antibody to attract and separate the analyte from the solution. Thus, the affinity of the antibody for the analyte limits the efficiency of analyte capture from the test sample, and together with the heterogeneity of the reaction mixture (i.e., soluble analyte and immobilized capture antibody on a solid phase) can lead to poor reaction kinetics and poor separation during the brief "pass-through" of sample through the solid phase.

In the present invention, both the capture and indicator reagents are soluble and are dispersed in a fluid test sample to form an homogeneous reaction mixture. The homogeneous reaction mixture is believed to provide an enhanced opportunity for complexing the charged capture antibody as well as the labeled antibody to an antigen analyte. The dispersion of analyte and charged capture antibody in the solution is believed to increase both the reaction kinetics and the probability of analyte/antibody binding as compared to the binding during the "pass-through" technique.

When the complex of charged capture reagent and analyte (and/or indicator reagent) is passed through the oppositely charged solid phase, the ionic attraction of the oppositely charged species governs the efficiency of the separation of the complex from the solution. The ionic attraction can be selected to provide a greater attraction than the attraction of antibody for antigen in other separation techniques, particularly when multiple polycationic and polyanionic species are used. A further advantage is that the "ion-capture" technique minimizes the nonspecific adsorption of interfering substances onto the solid phase material, thereby offering improved accuracy of analysis.

### DETAILED DESCRIPTION OF THE INVENTION

### I. GENERAL

The assay methods and reagents of the present invention can be used in a variety of immunoassay formats. The present invention, however, is not limited to immunoreactive assays. Any assays using specific binding members can be performed. A "specific binding member", as used herein, is a member of a specific binding pair, i.e., two different molecules where one of the molecules through chemical or physical means specifically binds to the second molecule. Therefore, in addition to antigen and antibody specific binding pairs, other specific binding pairs can include biotin and avidin, carbohydrates and lectins, complementary nucleotide sequences, effector and receptor molecules, enzyme cofactors and enzymes, enzyme inhibitors and enzymes, and the like. Furthermore, specific binding pairs can include members that are analogs of the original specific binding member, for example an analyte-analog. Immunoreactive specific binding members include antigens, haptens, antibodies, and complexes thereof including those formed by recombinant DNA methods.

In a sandwich assay, a soluble capture reagent is used which includes a specific binding member which has been bound to a charged substance such as an anion or cation. The ionic species can be a monomer or a polymer. If the specific binding member is an immunoreactant it can be an antibody, antigen, or complex thereof, specific for the analyte of interest, and if an antibody is used, it can be a monoclonal or polyclonal antibody, an antibody fragment, or a recombinant antibody, as well as a mixture thereof, or a mixture of an antibody and other specific binding members. The capture reagent is contacted with a test sample, suspected of containing the analyte, and an indicator reagent comprising a second specific binding member that has been labeled with a signal generating compound (e.g., an enzyme, or a fluorescent or chemiluminescent compound.) The reagents can be mixed simultaneously or added sequentially, either singly or in combination. A binding reaction results in the formation of a capture reagent/analyte/indicator reagent complex. The assay also comprises the step of separating the resultant soluble complex from the excess reagents and test sample by using a solid phase material that is either oppositely charged with respect to the capture reagent or which retains an oppositely charged substance, such as an anionic or cationic polymer. The oppositely charged solid phase material attracts and attaches to the capture reagent/analyte/indicator reagent complex through the interaction of the opposite charges. The solid phase material can be any suitable material, preferably a porous material, such as paper, a glass microfiber material, and the like.

The complex retained on the solid phase material is detected by examining the solid phase for the indicator reagent. If analyte is present in the sample, then label will be present on the solid phase material. The amount of label on the solid phase is proportional to the amount of analyte in the sample. An enzyme label can be detected visually or measured spectrophotometrically following the addition of an enzyme substrate. Or, the label can be detected by the measurement of fluorescence, chemiluminescence, radioactive energy emmissions, etc., depending on the label used.

"Analyte", as used herein, is the substance to be detected or separated from the sample using the present invention. The analyte can be any substance for which there exists a naturally occuring specific binding member (e.g., an antibody) or for which a specific binding member can be prepared. Thus, an analyte is a substance that can bind to one or more specific binding members in an assay. "Analyte" also includes any antigenic substances, haptens, antibodies, and combinations thereof. As a member of a specific binding pair the analyte can be detected by means of naturally occuring specific binding partners such as the use of intrinsic factor protein in the capture and/or indicator reagents for the determination of vitamin B₁₂ or the use of a lectin in the capture and/or indicator reagents for the determination of a carbohydrate. The analyte can include a protein, a peptide, an amino acid, a hormone, a steroid, a vitamin, a drug including those administered for therapeutic purposes as well as those administered for illicit purposes, a bacterium, a virus, and metabolites of or antibodies to any of the above substances.

The present invention also can be used to conduct a competitive assay. In a competitive configuration, the soluble capture reagent again includes a specific binding member which has been bound to a charged substance, such as an anionic or cationic polymer. The capture reagent is contacted with both test sample and an indicator reagent that includes an analyte or analyte-analog which has been labeled with a signal generating compound. A reaction occurs resulting in the formation of soluble complexes of (1) capture reagent/test sample analyte and (2) capture reagent/indicator reagent.

The soluble complexes are removed from the excess reagents and test sample by contacting the reaction mixture with the oppositely charged solid phase material. The complexes are retained on the solid phase through its attraction of the oppositely charged capture reagent. The complexes retained on the solid phase are detected via the label on the indicator reagent. In the competitive assay, the amount of label on the solid phase is inversely proportional to the amount of analyte in the sample. Thus, a positive test sample will generate a negative signal.

For example, in an assay for theophylline, an anti-theophylline antibody (either monoclonal or polyclonal) can be conjugated with an anionic substance to form a soluble capture reagent, and a competition for binding to that antibody can be established between the soluble labeled theophylline (i.e., indicator reagent) and the unlabeled theophylline of the test sample. After incubation, the homogeneous mixture can be passed through a cation-coated porous solid phase material. The attraction between the oppositely charged ionic species of the capture reagent and the solid phase separates the immunocomplex from the reaction mixture. The signal from the indicator reagent can then be detected. In this case, increased theophylline levels in the test sample will result in decreased signal generation associated with the solid phase.

Similarly, the present invention can be used in a sandwich assay to form an immunocomplex of capture reagent/analyte/indicator reagent and variations thereof, such as an indirect immunoassay with the formation of a complex of capture reagent/analyte/anti-analyte antibody/indicator reagent. Thus, an indicator reagent can comprise a specific binding partner either for the analyte in a sandwich assay, for the capture reagent in a competitive assay, or for an ancillary specific binding member, which itself is specific for the analyte, in an indirect assay. Similarly, a capture reagent can comprise a specific binding partner either for the analyte in a sandwich assay, for the analyte and indicator reagent in a competitive assay, or for an ancillary specific binding member, which itself is specific for the analyte, in an indirect assay.

The present invention also can be used solely for separating a substance from a solution. For example, the capture reagent and solid phase material can be used without the indicator for the purpose of separating an analyte from a test sample. Furthermore, the capture reagent can be contacted with a soluble second charged substance which is oppositely charged with respect to the charged substance of the capture reagent. The second charged substance is not retained on the solid phase material prior to contacting the sample to the solid phase material, but it attracts and attaches to the capture reagent such that the resultant assay complexes are retained on the solid phase.

### II. REAGENTS AND MATERIALS

### a) INDICATOR REAGENT

The indicator reagent comprises a label conjugated to a specific binding member. The indicator reagent produces a detectable signal at a level relative to the amount of an analyte in the test sample. In general, the indicator reagent is detected or measured after it is captured on the solid phase material, but the unbound indicator reagent also can be measured to determine the result of an assay.

In addition to being either an antigen or an antibody member of a specific binding pair, the specific binding member of the indicator reagent can be a member of any specific binding pair including either biotin or avidin, a carbohydrate or a lectin, a complementary nucleotide sequence, an effector or a receptor molecule, an enzyme cofactor or an enzyme, an enzyme inhibitor or an enzyme. An immunoreactive specific binding member can be an antibody, antigen, or antibody/antigen complex that is capable of binding either to the analyte as in a sandwich assay, to the capture reagent as in a competitive assay, or to an ancillary specific binding member as in an indirect assay. If an antibody is used, it can be a monoclonal antibody, polyclonal antibody, antibody fragment, recombinant antibody, a mixture thereof, or a mixture of an antibody and other specific binding members. The details of the preparation of such antibodies and their suitability for use as specific binding members are well known and will not be repeated here.

The label of the indicator reagent is capable of producing a measurable signal detectable by external means. The various labels can include chromogens, catalysts, fluorescent compounds, chemiluminescent compounds, radioactive labels, and direct visual labels. The selection of a particular label is not critical, but it will be capable of producing a signal either by itself or in conjunction with one or more additional substances.

A variety of different indicator reagents can be formed by varying either the label or the specific binding member. In one embodiment of the present invention the indicator reagent comprises a monoclonal antibody bound to an enzyme label, for example, alkaline phosphatase bound to an anti-carcinoembryonic antigen antibody fragment.

### b) CAPTURE REAGENT

The capture reagent of the present invention has two basic components. The first component is a specific binding member, specific either for the analyte as in a sandwich assay, for the indicator reagent and analyte as in a competitive assay, or for an ancillary specific binding member, which itself is specific for the analyte, as in an indirect assay. The second component is a charged substance conjugated to the first component. The conjugation of the components is essentially irreversible and can include covalent mechanisms.

The charged substances can include anionic and cationic monomers or polymers. For example, anionic polymers include polyglutamic acid (PGA), anionic protein or derivitized protein such as albumin, anionic saccharides such as heparin or alginic acid, polyaspartic acid, polyacrylic acid, and polyamino acids having a net negative charge at an appropriate pH (such as a pH in the range of 4 to 10.) Furthermore, the specific binding member can be joined to more than one charged monomer or polymer to increase the net charge associated with the capture reagent.

The specific binding member of the capture reagent can be any molecule capable of specifically binding with another, just as in the indicator reagent specific binding members. The specific binding member of the capture reagent can be an immunoreactive component such as an antibody, antigen, or antibody/antigen complex. If an antibody is used, it can be a monoclonal antibody, polyclonal antibody, antibody fragment, recombinant antibody, a mixture thereof, or a mixture of an antibody and other specific binding members.

### c) SOLID PHASE MATERIAL

The present invention also includes a solid phase material. The solid phase material can be chosen for its intrinsic charge and ability to attract the capture reagent, e.g., methylated wool, nylons, and special glasses having a positive charge. Alternatively, the solid phase material can retain an additional charged substance that is oppositely charged with respect to the charged substance of the capture reagent. For example, an anionic substance can be bound to the capture reagent, and a cationic substance retained on the solid phase material, or vice versa.

Once complex formation occurs, the solid phase is used as a separation mechanism: the homogeneous reaction mixture is contacted with the solid phase material, and the newly formed complex(es) are retained on the solid phase material through the attraction of the opposite charges of the solid phase material and the capture reagent.

An assay device for the present invention can have many configurations, several of which are dependent upon the material chosen as the solid phase material. The solid phase material can include any suitable porous material. By "porous" is meant that the material is one through which fluids can flow and can easily pass. In the present invention, the solid phase material can include a fiberglass, cellulose, or nylon pad for use in a pour and flow-through assay device having one or more layers containing one or more of the assay reagents; a dipstick for a dip and read assay; a test strip for chromatographic (e.g., paper) or thin layer chromatographic (e.g., nitrocellulose) techniques in which one or all of the reagents are contained in separate zones of a single strip of solid phase material; or other porous material well known to those skilled in the art. The solid phase material, however, is not limited to porous materials. The solid phase material can also comprise 1/4 inch (0.64 cm) beads, magnetic beads, latex particles, a glass test tube, or any other material which has an intrinsic charge or which can retain a charged substance.

Natural, synthetic, or naturally occurring materials that are synthetically modified, can be used as a solid phase material including polysaccharides, e.g., cellulose materials such as paper and cellulose derivatives such as cellulose acetate and nitrocellulose; silica; inorganic materials such as deactivated alumina, diatomaceous earth, MgSO₄, or other inorganic finely divided material uniformly dispersed in a porous polymer matrix, with polymers such as vinyl chloride, vinyl chloride-propylene copolymer, and vinyl chloride-vinyl acetate copolymer; cloth, both naturally occuring (e.g., cotton) and synthetic (e.g., nylon); porous gels such as silica gel, agarose, dextran, and gelatin; polymeric films such as polyacrilamide; and the like. The solid phase material should have reasonable strength or strength can be provided by means of a support, and it should not interfere with the production of a detectable signal.

Preferred solid phase materials include a porous fiberglass material, such as a "Whatman 934-AH"™ filter paper, which has a nominal thickness of 0.33 mm, or the disposable IMx™ wedge and TestPack™ (fiber matrix) devices of Abbott Laboratories (Abbott Park, IL, 60064). The thickness of such material is not critical, and will be a matter of choice, largely based upon the properties of the sample or analyte being assayed, such as the fluidity of the test sample.

To change or enhance the intrinsic charge of the solid phase material, a charged substance can be coated directly to the material or onto microparticles which are then retained by a solid phase base material. Alternatively, microparticles alone can be used as the charged solid phase material. One possible charged substance is a polymeric cation which is retained by the solid phase material and which will attract and retain the oppositely charged species which becomes a part of the assay complex via the capture reagent.

A wide variety of proprietary polycations are available including GafQuat™ (GAF Corporation, Wayne, NJ, 07470), diethylaminoethyl-dextran (Sigma Chemical Company, St. Louis, Mo.), and water soluble cellulose derivatives such as Celquat™ L-200 and Celquat™ H-100 (National Starch & Chemical Corporation, Bridgewater, NJ, 08807).

### d) ANCILLARY MATERIALS

Although it is not critical to the present invention, the charged substance also can be coated onto particles, e.g., "beads" or "microparticles". These particles can serve as the solid phase, by being retained in a column or being suspended in the mixture of soluble reagents and test sample, or the particles themselves can be retained and immobilized by a solid phase base material. By "retained and immobilized" is meant that the particles, once on the solid phase material, are not capable of substantial movement to positions elsewhere within the material. The particles can be selected by one skilled in the art from any suitable type of particulate material composed of polystyrene, polymethylacrylate, polypropylene, latex, polytetrafluoroethylene, polyacrylonitrile, polycarbonate, or similar materials. The size of the particles is not critical, although it is preferred that the average diameter of the particles be smaller than the average pore size of the solid phase base material being used.

"Ancillary specific binding member" is used to refer to any member of a specific binding pair which is used in an assay or separation procedure in addition to the specific binding members of the capture reagent and the indicator reagent. One or more ancillary specific binding members can be used in an assay, as demonstrated in the Examples.

### EXAMPLES

The following Examples illustrate preferred ways of making the novel materials of the present invention and performing assay procedures using those materials. The Examples, however, are intended only to be illustrative, and are not to be construed as placing limitations upon the scope of the invention, which scope is defined solely by the appended claims.

### A. Sandwich assay for Carcinoembryonic antigen

### Example 1: Preparation of a capture reagent

The following sequence of steps describes the chemistry employed for the preparation of an antibody/polyglutamic acid (PGA) conjugate, i.e., an antibody/anionic polymer capture reagent.

### a. Preparation of a traceable anionic polymer

The sodium salt of PGA (one gram; 7.14 x 10⁻⁵ mole; average molecular weight 14,000; Sigma Chemical Company, St. Louis, Mo.) was converted to 3-(2-pyridyldithio) propionyl-PGA (PDP-PGA) by the method of Tsukada, et al. (JNCI; 73; 721-729, 1984) with the following procedural modifications. The PDP-PGA was not reduced to the free sulfhydril prior to the thiopropyl sepharose 6B isolation. Instead, the PDP-PGA was dissolved in 0.1 M Na phosphate and 1 mM EDTA (pH 6.5) and stirred with thiopropyl sepharose 6B (60 ml; 30 grams; Pharmacia Chemicals, Uppsala, Sweden). After dialysis and lyophilization, a 24% yield of the PDP-PGA conjugate was obtained (0.244 grams; 1.72 x 10⁻⁵ mole).

To ensure that the disulfide was maintained during the ensuing chemistries, the thiopyridyl group was exchanged for a 5-thio-2-nitrobenzoate (TNB) protecting group. A 100 mole excess of 1,4-dithiothreitol (molecular weight 154.2) was added to a solution of the PDP-PGA (20 mg; 1.42 x 10⁻⁶ mole) dissolved in 0.1 M sodium phosphate (4 ml; pH 7), and the reaction was run for one hour at 40° C. The mixture was diluted to ten milliliters with 5 mM sodium acetate, 0.14 M NaCI, and 1 mM EDTA (pH 5.5) and dialyzed in 2000 molecular weight cut off (MWCO) tubing against the dilution buffer. Dialysis was continued against distilled water, followed by lyophilization. The yield of thiopropyl-PGA (HS-PGA) was 13.5 mg. The HS-PGA (13.5 mg) was dissolved in 0.1 M sodium phosphate (pH 7; 9.6 x 10⁻⁷ mole) and reacted with a 10 mole excess of 5,5' dithiobis (2-nitrobenzoic acid) (DTNB) for one hour at room temperature. This mixture was diluted to ten milliliters with 0.1 M sodium phosphate (pH 7) and dialyzed in 2000 MWCO tubing against the dilution buffer. Dialysis was continued against distilled water and was followed by lyophilization to produce 5-(2-nitrobenzoic dithio) propionyl-PGA (TNB-PGA; 8.5 mg; 6.07 x 10⁻⁷ mole).

To trace the number of anionic polymer molecules attached to each capture reagent antibody, the TNB-protected PGA was then labeled with an ethylenediamine derivative of fluorescein. The TNB-PGA was loaded with an ethylenediamine derivatized fluorescein (EDA-FI; molecular weight 532) by dissolving TNB-PGA (8.5 mg) in dry N-N dimethyl-formamide (2 ml), treating with a 90 mole excess of N-methylmorpholine (molecular weight 101.15), lowering the temperature to 0° C, and adding a 90 mole excess of isobutylchloroformate (molecular weight 136.58). This reaction was run at 0° C for one hour. The mixture was warmed to room temperature, a 30 mole excess of EDA-FI was added, and the reaction was run at room temperature with stirring overnight. The mixture was diluted to ten milliliters with 0.1 M sodium phosphate (pH 7) and dialyzed in 2000 MWCO tubing against the dilution buffer. Dialysis was continued against distilled water and was followed by lyophilization to yield TNB-PGA/EDA-FI conjugate (7.8 mg; 5.6 x 10⁻⁷ mole).

The TNB group was removed by dissolving the TNB-PGA/EDA-FI (7.8 mg) in 0.1 M sodium phosphate (3 ml; pH 7) and treating with a 100 mole excess of 1 ,4-dithiotheritol for one hour at 40° C. The reaction was monitored for a shift of a 334 nm to a 412 nm peak on a UV/VIS spectrophotometer. The material was diluted to ten milliliters with distilled water and dialyzed in 2000 MWCO tubing against distilled water. Upon lyophilization, thiopropyl-PGA/EDA-FI (HS-PGA/EDA-FI; 8.4 mg) was obtained. At this point, a UV/VIS scan was taken to determine the number of fluoresceins per PGA molecule (i.e., loading). A value of 0.81 fluoresceins per PGA was calculated for this preparation.

### b. Antibody activation

The monoclonal antibody, an anti-CEA antibody was maleimide activated per the method of Tuskada, et al. (JNCI: 73; 721-729, 1984) with the following exceptions. The antibody concentration was one mg/ml, and a 150 mole excess of N-succinimidyl m-(N-maleimido) benzoate (SMBE, molecular weight 314.3; Sigma) was used. It was determined experimentally that a 150 mole excess was necessary to introduce between three and five maleimide groups to the anti-CEA antibody. Clean-up was performed using the Meares, et al. centrifuge method (Analytical Biochemistry: 1 142; 68-78, 1984) with Sephadex™ G-50/80 (Sigma) in three milliliter syringe columns. The number of maleimides per antibody was determined using the titration method of Liu, et al., (Biochemistry: 18; 690-696, 1979). It was found that 4.6 maleimides were introduced per antibody during this antibody activation.

The thiopropyl-protected, fluorescein-labeled PGA of Example A.1.a. was then reacted with the maleimide derived antibody to yield the antibody/PGA conjugate appropriate for a carcinoembryonic antigen ion-capture immunoassay. The maleimide-activated antibody (1.0 mg; 6.25 x 10⁻⁹ mole) in 0.1 M sodium phosphate (1 to 2 ml; pH 7) was pH adjusted to 6.5 with 1 N HCl. Then, a 10 mole excess of HS-PGA/EDA-FI (approximately 1 mg) in 0.1 M sodium phosphate (100 µl) was added to the activated antibody preparation. The conjugation was run overnight with gentle stirring at room temperature. The mixture was diluted to ten milliliters in 0.1 M sodium phosphate (pH 7) and dialyzed in 50,000 MWCO tubing against 0.001 M Na phosphate (pH 7) followed by lyophilization. The dry material was redissolved in distilled water (0.25 ml) and high performance liquid chromatography fractioned for the largest peak at A280. The chromatography was performed using a Bio-Sil TSK250™ (Bio-Rad Laboratories, Richmond, California) 300 mm x 7.5 mm column, eluted at one milliliter/minute with 50 mM sodium sulfate, 20 mM sodium phosphate, and 0.3 M NaCl (pH 6.8).

The largest peak was assayed for protein content using Bio-Rad's Bradford assay with a bovine IgG standard. The peak contained 95.5 µg/ml protein equating to 5.97 x 10⁻⁷ molar protein (IgG molecular weight 160,000). By scanning the UV/VIS and taking the absorbance at 494 nm, it was determined that this fraction also contained 2.12 x 10⁻⁶ molar fluorescein. The equation of the molar fluorescein gave 3.6 fluoresceins per antibody molecule. Knowing that there were 0.81 fluoresceins per PGA molecule, this equated to 4.4 PGA molecules conjugated to each antibody. The peak fraction was frozen and subsequently used in the assay.

An important aspect of the above described chemistries is that there exists but a single site of attachment between each polymeric anion and the antibody. The solitary covalent link between the two circumvents the potential intermolecular and intramolecular crosslinking that could occur if a polymeric anion bearing multiple activated groups were employed.

As an alternative to the above capture reagent example, a cationic derived antibody could also be formed for use in conjunction with an anionic solid phase material.

### Example 2: Preparation of the solid phase material

The solid phase fibrous matrix of a disposable IMx™ wedge was coated with a polymeric quaternary compound to give the solid phase material a positive charge. Celquat™ L-200, a water soluble cellulose derivative, was used. A 1% aqueous solution of Celquat™ L-200 (50 µl) was applied to the solid phase material, followed by a wash of diluent containing 300 mM NaCl, 50 mM Tris and 0.1% NaN₃ (75 µl; pH 7.5).

### Example 3: Indicator reagent

The indicator reagent consisted of a conjugate of alkaline phosphatase and anti-CEA antibody fragment, which binds to a different epitope than the antibody specified in the capture reagent of Example 1.b. above. The alkaline phosphatase-labeled anti-CEA antibody fragment was in a buffer containing: 50 mM Tris, 50 mM NaCl, 1 mM MgCl₂, 0 0.1 mM ZnCl₂, 5 mM sodium tartrate, 0.5% calf skin gelatin, and 3% mouse serum.

### Example 4: Immunoassay Protocol - Determination of CEA

The ion-capture immunoassay protocol included the use of the solid phase material, prepared as described in Example A.2. The indicator reagent of Example A.3 (70 µl) was placed into a reaction well. Then, buffered capture reagent as prepared in Example A.1 (20 µl of anti-CEA/PGA conjugate in a buffer of 50 mM Na₂SO₄, 20 mM sodium phosphate, and 300 mM NaCl at pH 6.8) was added to the well. A 35 µl specimen containing CEA was added to the well, and the homogeneous immunoreaction mixture was incubated for 20 minutes at 34.5° C. Four different specimens were run in the assay, each of which was a CEA calibrator from the Abbott Laboratories CEA enzyme immunoassay kit. An aliquot of each reaction mixture (100 µl) was then applied to the quat-treated solid phase material, followed by three 75 µl washes of diluent. Finally, an enzyme substrate (70 µl; 1 .2 mM 4-methylumbelliferyl-phosphate in a solution of 100 mM AMP, 1 mM MgCl₂, 0.1% NaN₃, and 4 mM tetramisole at pH 10.3) was added at 34.5° C for reaction with the indicator reagent, and the resulting rate of fluorescence was measured. The dose-response results of the assay are shown in Table 1.

**TABLE 1**

| CEA Ion-capture Sandwich Assay Capture reagent: PGA/anti-CEA antibody conjugate Indicator reagent: alkaline phosphatase-labeled anti-CEA antibody fragment | |
|---|---|
| CEA (ng/ml) | Rate (counts/sec/sec) |
| 0 | 37 |
| 4 | 170 |
| 30 | 931 |
| 80 | 2398 |

### B. Competitive inhibition assay of mouse immunoglobulin

Part B illustrates a complex and a method of forming it according to the invention.

### Example 1: Preparation of a capture reagent

A protein-A affinity purified mouse monoclonal immunoglobulin G was coupled to negatively charged PGA using a water-soluble carbodiimide reagent (1-ethyl-3-(3-dimethylamino-propyl) carbodiimide; EDCI) according to the following procedures.

Fluorescein-labeled PGA (10 mg; FI-PGA) was added to an ice-cold solution of the antibody (4.8 mg/ml) in phosphate-buffered saline (PBS; 75 mM KH₂PO₄ and 300 mM NaCl at pH 7.2). To that solution was added a freshly prepared ice-cold solution of EDCI (100 µl; 10 mg/ml), and the resultant reaction mixture was allowed to warm to room temperature with continuous stirring for 2.5 hours. An additional freshly prepared ice-cold solution of EDCI (50 µl;100 mg/ml) was then added to the reaction mixture with rapid stirring. The reaction mixture was stirred for another 1.5 hours. The mixture was then fractionated by gel filtration chromatography using a Spherogel™ TSK-3000SWG column (2.15 cm x 30 cm) fitted with a Spherogel™ TSK-G guard column (2.15 cm x 7.5 cm; Beckman Instruments, Inc., Fullerton, CA, 92634). The column was eluted with PBS at a flow rate of five milliliters/minute. The PGA/antibody ratio of these pools was determined by quantitating the fluorescence in the FI-PGA conjugates of the antibody. The results are shown in Table 2.

**TABLE 2**

| PGA/anti-CEA antibody conjugates prepared using EDCI | | |
|---|---|---|
| Pool | Peak Molecular Weight | PGA/antibody |
| I | 420,000 | 3.8 |
| II | 280,000 | 4.1 |
| III | 220,000 | 5.5 |

### Example 2: Preparation of the solid phase material

The solid phase material was coated with a polymeric quaternary ammonium compound (Gafquat™ 755N; GAF Corporation). An aqueous solution of 0.5% Gafquat™ (50 µl) was applied to the surface of the material, followed by a water wash (75 µl).

### Example 3: Binding of the indicator reagent to the capture reagent

The indicator reagent, an alkaline phosphatase conjugate of sheep anti-mouse immunoglobulin (Jackson ImmunoResearch Laboratories, Inc.; West Grove, PA, 19390), was diluted in Tris-buffered saline containing 1% fish gelatin [25 mM Tris (hydroxymethyl) aminomethane and 100 mM NaCI, pH 7.5]. The capture reagent of PGA/mouse monoclonal antibody conjugate (Pool I of Example B.1) was similarly treated. Two hundred microliters of each reagent was added to a series of test tubes which were then incubated at 37° C for 30 minutes. An aliquot of the reaction mixture (75 µl) was applied to the quat-treated materials (of Example B.2), immediately followed by three 150 µl washes of Tris-buffered saline. Finally, an enzyme substrate (70 µl of 1.2 mM 4-methylumbelliferylphosphate in a solution of 100 mM AMP, 1mM MgCl₂, 0.1% NaN₃, and 4 mM tetramisole; pH 10.3) was added to the materials at 32.7° C, and the resulting rate of fluorescence was measured. The results of the experiment are summarized in Tables 3 and 4.

**TABLE 3**

| Dose response of capture reagent/indicator reagent binding | |
|---|---|
| PGA/antibody^{*} (µg/ml) | Rate of fluorescence (counts/sec/sec) |
| 10 | 1559 |
| 1 | 816 |
| 0.1 | 179 |
| 0.01 | 70 |
| 0 | 36 |

| | |
|---|---|
| ^{*}The initial concentrations of PGA-coupled-antibody before mixing with a 1000-fold diluted alkaline phosphatase-labeled sheep anti-mouse immunoglobulin. | |

**TABLE 4**

| Dose response of indicator reagent/capture reagent^{*} binding | |
|---|---|
| Indicator reagent titer^{**} | Rate of fluorescence (counts/sec/sec) |
| 10² | 5062 |
| 10³ | 796 |
| 10⁴ | 93 |
| 10⁵ | 10 |
| 10⁶ | 5 |

| | |
|---|---|
| ^{*}The initial concentration of PGA-coupled-antibody before mixing with alkaline phosphatase-labeled sheep anti-mouse immunoglobulin was five µg/ml. | |
| ^{**}The indicator reagent titer is the reciprocal of the dilution of the reagent stock. | |

### Example 4: Competitive inhibition assay for mouse IgG

The capture reagent and indicator reagent were prepared as in Example B.3. All of the reagents were diluted in Tris-buffered saline containing 1% fish gelatin. The indicator reagent was diluted 1000-fold from the stock solution, and the capture reagent was diluted to ten µg/ml. In a series of test tubes, 150 µl each of appropriately diluted indicator reagent, capture reagent, and mouse monoclonal antibody were mixed. The mixtures were incubated at 37° C for 30 minutes. Aliquots of the mixtures (75 µl) were applied to the quat-treated materials (of Example B.2), immediately followed by three 150 µl washes of Tris-buffered saline. An enzyme substrate (70 µl of 1.2 mM 4-methylumbelliferylphosphate in a solution of 100 mM AMP, 1mM MgCl₂, 0.1% NaN₃, and 4 mM tetramisole; pH 10.3) was then added to the solid phase material at 32.7° C, and the resulting rate of fluorescence was measured. The results of this example illustrating a competitive inhibition assay for mouse IgG are shown in Table 5.

**TABLE 5**

| Inhibition of indicator reagent binding due to mouse monoclonal antibody Capture reagent: PGA/mouse monoclonal lgG conjugate Indicator reagent: alkaline phosphatase-sheep anti-mouse immunoglobulin conjugate | |
|---|---|
| Mouse IgG (µg/ml) | Rate of fluorescence (counts/sec/sec) |
| 0 | 110 |
| 3.3 × 10⁻³ | 106 |
| 3.3 × 10⁻² | 98 |
| 3.3 × 10⁻¹ | 67 |
| 3.3 | 36 |
| 33 | 10 |

### C. Sandwich assay for human chorionic gonadotropin

### Example 1: Preparation of the capture reagent

A highly negatively charged albumin derivative was prepared and coupled to anti-hCG antibodies to form the capture reagent according to the following procedures.

### a. Modification of rabbit serum albumin to form a neaativelv charged protein derivative

Rabbit serum albumin (RSA) was extensively succinylated and coupled with paraazobenzenesulfonate by the procedure of Jou, et al., (Methods in Enzymology: Vol. 92, Part E; 257-276, Academic Press, 1983). Two per cent RSA in phosphate-buffered saline (PBS, 14 ml, pH 8) was mixed with 5% succinic anhydride in para-dioxane (2.28 ml). The pH was maintained at 8 by the addition of 1 N NaOH. The reaction mixture was stirred at room temperature for 30 minutes. Hydroxylamine hydrochloride was added (0.6 g) and the pH of the solution was adjusted to 9.5 by adding an appropriate amount of 5 N NaOH. The mixture was then dialyzed against water. The resultant SUC₆₅-RSA was coupled to para-azobenzenesulfonate according to the following reactions.

A suspension of para-azobenzenesulfonic acid (0.15 mmole, 26 mg) in 1 N HCI (0.8 ml) was cooled in an ice bath and treated with 1 N NaNO₂ (0.2 ml) for 30 minutes with rapid stirring. The resultant diazonium salt solution was added by drops to the ice cooled SUC₆₅-RSA solution with rapid stirring. The pH of the reaction mixture was maintained at 11 by the addition of 1 N NaOH. The dark red reaction mixture was stirred and allowed to warm to room temperature for one hour before it was extensively dialyzed against water. The resultant Sp-SUC₆₅-RSA anionic derivatized protein was kept refrigerated until used.

### b. Preparation of anti-hCG F(ab')₂ fragments

Anti-hCG F(ab′)₂ fragments were prepared according to the method of Nisonoff, et al., (Arch. Biochem. Biophy.: 89; 230-244, 1960) from affinity purified goat anti-hCG antibodies. A portion of affinity purified antibody solution in phosphate buffered saline (pH 7.2) was acidified to pH 4 by adding acetic acid. The preferred concentration of antibodies at this point was one mg/ml. Pepsin was added to reach a final concentration of 20 µg/ml. The mixture was incubated at 37° C overnight. The reaction was stopped by adding 6 N NaOH to bring the reaction mixture to a pH of 7.5. The digested antibody fragments solution was concentrated to 20 mg/ml. The F(ab')₂ fragments were purified by get-filtration high performance liquid chromatography using a Spherogel™ TSK-3000SWG column (2.15 cm x 30 cm) fitted with a Spherogel™ TSK-G guard column (2.15 cm x 7.5 cm).

### c. Preparation of anti-hCG TNB-Fab' fragments

Anti-hCG Fab' fragments were prepared and derivatized into a thiol-reactive form according to a modification of the methods of Parham, et al., (J. Immunol. Method.: 53: 133-173, 1982) and Brennan, et al., (Science: 229: 81-83, 1985). With stirring, a solution (158 µl) of 0.1 M NaAsO₂ containing 20 mM EDTA was added to 1.28 ml of goat F(ab′)₂ (goat anti-human chorionic gonadotropin antibody fragment, 16 mg/ml) containing trace ¹²⁵I-F(ab′)₂ in PBS. The reductive cleavage reaction was started by adding 0.1 M cysteine-HCI (158 µl). The reaction mixture was overlayed with nitrogen and incubated with stirring at 37° C for one hour. The reaction was then quenched by adding 19 mg of 5,5'-dithiobis-(2-nitrobenzoic acid). After stirring overnight at room temperature, the mixture was chromatographed on a PD-10™ column (Pharmacia Inc., Piscataway, NJ) preequilibrated with PBS, and then chromatographed on a size exclusion high performance liquid chromatography column [Spherogel™ TSK-2000SWG column (2.15 cm x 30 cm) fitted with a Spherogel™ TSK-G guard column (2.15 cm x 7.5 cm)]. The purified thionitrobenzoate derivative of Fab' (TNB-Fab') was concentrated to 7.9 mg/ml using a CX-10 ultrafiltration unit (Millipore Corp., Bedford, MA).

### d. Coupling of anti-hCG TNB-Fab' fragments to Sp-SUC₆₅-RSA

A solution of 1 M dithiothrietol (DTT; 86 µl) was added to a solution (4.2 ml) containing Sp-SUC₆₅-RSA (2.2 mg/ml) in 37.5 mM sodium phosphate, 150 mM NaCI, and 2 mM EDTA (pH 6.8). The mixture was incubated at 37° C for three hours and then at room temperature overnight. The resulting reaction mixture was chromatographed on a 2.5 cm x 20 cm column packed with Sephadex™ G-25 (Pharmacia Inc.) and preequilibrated with 75 mM sodium phosphate, 300 mM NaCI, and 2 mM EDTA (pH 6.8). A two milliliter portion of the pooled fractions of reduced Sp-SUC₆₅-RSA (0.48 mg/ml) was mixed with anti-hCG TNB-Fab' (0.15 ml; 7.9 mg/ml). The mixture was stirred at room temperature overnight. The reaction mixture was then treated with 100 mM iodoacetic acid (107 µl) and stirred for one hour at room temperature. The Fab'-Sp-SUC₆₅-RSA conjugate was purified by size exclusion high performance liquid chromatography using a Spherogel™ TSK-3000SWG column (2.15 cm x 30 cm) fitted with a Spherogel™ TSK-G guard column (2.15 cm x 7.5 cm).

### e. Coupling of anti-hCG antibodies to Sp-SUC₆₅-RSA

A solution (27 µl) of 30 mM succinimidyl 4-(N-maleimido-methyl)-cyclohexane-1-carboxylate in N,N-dimethylformamide was added to 2.25 ml of affinity purified goat anti-hCG antibody (3 mg/ml) in PBS. The resulting reaction mixture was stirred for one hour at room temperature and then chromatographed on a PD-10™ column preequilibrated with 75 mM sodium phosphate, 300 mM NaCI, and 2 mM EDTA (pH 6.8). A 1.8 ml portion of the pooled fractions of modified antibodies (1.6 mg/ml) was mixed with three milliliters of DTT-reduced Sp-SUC₆₅-RSA (0.48 mg/ml) of Example C.1.d. After stirring at room temperature overnight, the reaction was quenched by adding 100 mM iodoacetic acid (0.25 ml) and stirring at room temperature for one hour. The antibody-Sp-SUC₆₅-RSA conjugate was purified by size exclusion high performance liquid chromatography in the same manner as described in Example C.1.d.

### Example 2: Preparation of the indicator reagent

The indicator reagent consisted of an alkaline phosphatase-goat anti-hCG antibody conjugate in an assay buffer containing 25 mM Tris (hydroxymethyl) aminomethane, 100 mM NaCl, 1 mM MgCl₂, 0.1 mM ZnCl₂, 0.07% NaN₃, and 1% fish gelatin at pH 7.5.

### Example 3: Sandwich immunoassay for hCG

The ion-capture immunoassay protocol included the use of a solid phase material prepared as in Example B.2, an indicator reagent (alkaline phosphatase-goat anti-hCG antibody conjugate) as described in Example C.2, one of two different capture reagents (goat anti-hCG Fab′-Sp-SUC₆₅-RSA and goat anti-hCG IgG-Sp-SUC₆₅-RSA) as prepared in Example C.1 .d. and e., and a purified hCG standard solution. All reagents were appropriately diluted in the assay buffer. Equal volumes (750 µl) of the indicator reagent and hCG sample solution were placed in a series of test tubes. After incubation at 37° C for 30 minutes, a 125 µl aliquot of each incubated mixture was mixed in a separate tube with an equal volume of a capture reagent. The resulting mixtures were incubated for 30 minutes. The assay mixture (75 µl) was then added to each solid phase material. The solid phase materials were then washed three times with 150 µl amounts of washing buffer [25 mM Tris (hydroxymethyl) aminomethane, 100 mM NaCl, 1 mM MgCl₂, 0.1 mM ZnCl₂, and 0.07% NaN₃ at pH 7.5]. An enzyme substrate (70 µl of 1.2 mM 4-methylumbelliferylphosphate in a solution of 100 mM AMP, 1 mM MgCl₂, 0.1% NaN₃, and 4 mM tetramisole at pH 10.3) was then added to the solid phase materials. The resulting rate of fluorescence was measured at 32.7° C. The results of the experiment are summarized in Table 6.

**TABLE 6**

| hCG Ion-capture Sandwich Assay Comparing Different Capture Reagents Indicator reagent: hCG-specific goat IgG-alkaline phosphatase | | |
|---|---|---|
| | Rate of fluorescence (counts/sec/sec) hCG-specific capture reagents | |
| hCG (mIU/ml) | Goat IgG-Sp-SUC₆₅-RSA | Goat Fab'-Sp-SUC₆₅-RSA |
| 0 | 63 | 64 |
| 12.5 | 96 | 110 |
| 25 | 121 | 134 |
| 50 | 146 | 166 |
| 100 | 182 | 212 |

### Example 4: Indirect sandwich immunoassay for hCG

The indirect ion-capture immunoassay included the use of a solid phase material prepared as in Example B.2, an indicator reagent of alkaline phosphatase-sheep anti-mouse IgG conjugate (Jackson ImmunoResearch Laboratories, Inc.), a capture reagent of goat anti-hCG F(ab')₂-Sp-SUC₆₅-RSA as prepared in Example C.1.e., an ancillary specific binding member of mouse monoclonal anti-hCG antibodies (ImmunoSearch; Thomas River, NJ, 08753), and a purified hCG standard solution. The ancillary specific binding member was used to bind with the analyte and the indicator reagent. All reagents were appropriately diluted in the assay buffer. Equal volumes (150 µl) of the indicator reagent, hCG sample solution, and ancillary specific binding member were placed in a series of test tubes. After incubation at 37° C for five minutes, a 150 µl portion of capture reagent was added to each tube. The resulting mixtures were incubated for five minutes. The assay mixture (200 µl) was then added to each prepared solid phase material. The solid phase materials were then washed with washing buffer and treated with an enzyme substrate solution in the same manner as described in Example C.3. above. The resulting rate of fluorescence was measured at 32.7° C. The results of the assay are summarized in Table 7.

**TABLE 7**

| Ion-capture Indirect Sandwich Assay for hCG Capture reagent: goat anti-hCG F(ab')₂-Sp-SUC₆₅-RSA Indicator reagent: sheep anti-mouse IgG-alkaline phosphatase Ancillary specific binding member: mouse monoclonal anti-hCG antibody | |
|---|---|
| hCG (mIU/ml) | Rate of fluorescence (counts/sec/sec) |
| 0 | 13 |
| 1.5 | 18 |
| 3.3 | 27 |
| 6.3 | 40 |
| 12.6 | 70 |
| 25.0 | 112 |
| 50.0 | 230 |
| 100.0 | 443 |
| 200.0 | 732 |

### Example 5: Double indirect sandwich immunoassay for hCG

The ion-capture immunoassay protocol included the use of a solid phase material prepared as in Example B.2, an indicator reagent of alkaline phosphatase-sheep anti-mouse IgG conjugate (Jackson ImmunoResearch Laboratories, Inc.), an ancillary specific binding member of mouse monoclonal anti-hCG antibodies (ImmunoSearch; Thomas River, NJ, 08753), and a purified hCG standard solution. Additionally, the protocol used a second ancillary specific binding member of affinity purified goat anti-hCG antibodies and a capture reagent of rabbit anti-goat IgG-Sp-SUC₆₅-RSA. The capture reagent was prepared by coupling affinity purified rabbit anti-goat IgG (Cappel; Cochranville, PA, 19330) to Sp-SUC₆₅-RSA according to the procedure described in Example C.1.e. above. All reagents were appropriately diluted in the assay buffer. Equal volumes (100 µl) of the indicator reagent, hCG sample solution, and first ancillary specific binding member were placed in a series of test tubes. After incubation (37° C for ten minutes) the second ancillary specific binding member (100 µl) was added and the incubation was continued (at 37° C for an additional five minutes). Finally, capture reagent (100 µl) was added to each tube. The resulting mixtures were incubated for five minutes. The assay mixture (200 µl) was then added to each prepared solid phase material. The solid phase materials were then washed with washing buffer, treated with enzyme substrate solution, and measured for the rate of fluorescence in the same manner as described in Example C.4. above. The results of the assay are summarized in Table 8.

**TABLE 8**

| Ion-capture Double Indirect Sandwich Assay for hCG Capture reagent: rabbit anti-goat IgG-Sp-SUC₆₅-RSA Indicator reagent: sheep anti-mouse IgG-alkaline phosphatase Ancillary specific binding member: mouse monoclonal anti-hCG antibody Ancillary specific binding member: goat anti-hCG antibodies | | |
|---|---|---|
| | Rate of Fluorescence (counts/sec/sec) | |
| Goat anti-hCG (ng/ml) | hCG (40 mIU/ml) | Negative Control(0 mIU/ml) |
| 250 | 3499 | 36 |
| 150 | 3708 | 34 |
| 50 | 3543 | 33 |
| 25 | 3155 | 30 |

### D. Indirect assays - antiprogesterone and progesterone

### Example 1: Preparation of a capture reagent

The following sequence of steps describes the chemistry employed for the preparation of an antibody/polyglutamic acid conjugate.

### a. Conversion of PGA-sodium salt to the free acid form

The sodium salt of PGA (200 mg; 1.47 x 10⁻⁵ mole; average molecular weight 13,600; Sigma Chemical Company, St. Louis, Mo.) was stirred with a cation exchange resin (AG50W-X8; 13 grams; Bio-Rad, Richmond, CA) in 60 milliliters of water for three hours. The supernatant was decanted, filtered, and evaporated providing an 80% yield of the free acid form of PGA as a white powder (137 mg; average molecular weight 11,620).

### b. Preparation of ITC-PGA

To a solution of the free acid form of PGA (65 mg; 5.6 x 10⁻⁶ mole) in dimethylformamide (DMF; 2 ml) was added triethylamine (100 µl; 7.2 x 10⁻⁴ mole) and 1,3,3-phenylenediisothiocyanate (110 mg; 5.7 x 10⁻⁴ mole; Aldrich Chemical Company, Milwaukee, WI). After stirring overnight at room temperature. acetic acid (100 µl; 1.7 x 10⁻³ mole) was added, and the reaction mixture was then evaporated. Methylene chloride (25 ml) was added to the residue, and after stirring for two hours the mixture was filtered to yield the ITC-PGA as a white powder (101 mg).

### c. Preparation of PGA-labeled goat anti-mouse capture reagent

The ITC-PGA (295 µg; 2.5 x 10⁻⁸ mole; in 40 µl of 20% DMF/0.1 M scdium phosphate at pH 7.0) was added to a buffered solution of goat anti-mousa IgG (200 µg; 1.25 x 10⁻⁹ mole; Sigma Chemical Company; in 40 µl of 0.1 M sodium phosphate at pH 7) to form the PGA-labeled goat anti-mouse capture reagent. After stirring at room temperature for two days, 0.1 M Tris (20 µl; pH 7.4) was added and the resulting mixture was stored at 2 to 8° C until used.

### Example 2: Immunoassay for anti-progesterone antibody

The anti-progesterone antibody ion-capture immunoassay included the use of solid phase materials coated with a polymeric quaternary compound as described in Example A.2. A 60 µl sample was added to a reaction well. The samples consisted of a monoclonal anti-progesterone antibody at concentrations of 0, 5, 50, 100, 250, and 500 ng/ml in phosphate-buffered saline (PBS; 50 mM sodium phosphate, 99 mM NaCI, 0.1% NaN₃, at pH 7.4). Next, 20 µl of PBS were added to the reaction well, followed by 20 µl of the buffered indicator reagent, progesterone labeled with alkaline phosphatase (3 µg/ml in a Tris buffer of 50 mM Tris, pH 7.4, 150 mM NaCl, 1% NaN₃, 1 mM MgCl₂, 0.1 mM ZnCl_{2,} and 1% BSA). After incubating the mixture at 34.5° C for ten minutes, the capture reagent was added (20 µl; PGA-labeled goat anti-mouse antibody at a 1/100 dilution in PBS of the stock solution described in Example D.1.). The mixture was then incubated an additional ten minutes at 34.5° C. A 100 µl aliquot of the mixture was then applied to the solid phase material, followed by three 75 µl washes of diluent. Lastly, the enzyme substrate solution (70 µl; 1.2 mM 4-methylumbelliferylphosphate in a solution of 100 mM AMP, 1mM MgCl₂, 0.1% NaN₃, and 4 mM tetramisole at pH 10.3) was added to the solid phase, and the resulting rate of fluorescence was measured. The results of the assay are shown in Table 9.

**TABLE 9**

| Ion-capture Assay for Mouse Monoclonal Anti-progesterone Antibody Capture reagent: PGA-labeled goat anti-mouse antibody Indicator reagent: alkaline phosphatase-labeled progesterone | |
|---|---|
| Anti-progesterone(ng/ml) | Rate of fluorescence (counts/sec/sec) |
| 0 | 9 |
| 5 | 31 |
| 50 | 254 |
| 100 | 441 |
| 250 | 1191 |
| 500 | 2721 |

### Example 3: Indirect competitive immunoassay for progesterone

The solid phase material was prepared as in Example A.2. A 60 µl sample of various concentrations of progesterone in PBS was mixed with 20 µl of progesterone-labeled alkaline phosphatase indicator reagent (0.4 µg/ml in the Tris buffer of Example D.2.) and 20 µl of mouse anti-progesterone antibody as an ancillary specific binding member (0.3 µg/ml in PBS). After incubating the mixture at 34.5° C for ten minutes, 20 µl of of the PGA-labeled goat anti-mouse antibody capture reagent were added as described in Example D.2. above. The resulting mixture was incubated an additional ten minutes at 34.5° C. A 100 µl aliquot of the mixture was then applied to the solid phase material, followed by three washes of diluent. Lastly, 70 µl of the enzyme substrate solution (as in Example D.2.) was added to the solid phase, and the resulting rate of fluorescence was measured. The results of the assay are shown in Table 10.

**TABLE 10**

| Ion-capture Indirect Competitive Assay for Progesterone Capture reagent: PGA-labeled goat anti-mouse antibody Indicator reagent: alkaline phosphatase-labeled progesterone Ancillary specific binding member: mouse anti-progesterone antibody | |
|---|---|
| Progesterone(ng/ml) | Rate of fluorescence (counts/sec/sec) |
| 0 | 1203 |
| 1.88 | 277 |
| 3.75 | 145 |
| 7.5 | 67 |
| 15 | 30 |
| 30 | 16 |

The concepts of the present invention are applicable to various types of binding assays. It will be appreciated, however, that one skilled in the art can conceive of many other types of assays, including assays for analytes other than antigens or antibodies, to which the present inventive concepts can be applied. The embodiments described and the alternative embodiments presented are intended as examples rather than as limitations. Thus, the description of the invention is not intended to limit the invention to the particular embodiments disclosed, but it is intended to encompass all equivalents and subject matter as described above and as set forth in the following claims.

## Claims

1. A process for separating an analyte from a fluid sample. comprising:
a) providing
(1) a soluble capture reagent comprising a specific binding member conjugated to a first charged substance, wherein said specific binding member directly binds the analyte or indirectly binds the analyte by means of an ancillary specific binding member which is specific for the analyte, and
(2) an insoluble solid phase material comprising a second charged substance which is oppositely charged with respect to said first charged substance;
b) contacting the fluid sample with said capture reagent, whereby said capture reagent becomes bound to the analyte in the sample thereby forming a capture reagent/analyte complex; and
c) separating said capture reagent/analyte complex from the sample by contacting the sample with said solid phase material, whereby said solid phase material attracts and attaches to said first charged substance, thereby separating said capture reagent/analyte complex from the sample.

2. The process according to Claim 1, wherein said capture reagent indirectly binds the analyte by means of an ancillary specific binding member which is specific for the analyte.

3. A process for detecting an analyte in a fluid sample, comprising:
a) providing
(1) a soluble capture reagent, comprising a first specific binding member conjugated to a first charged substance, wherein said first specific binding member directly binds the analyte or indirectly binds the analyte by means of an ancillary specific binding member which is specific for the analyte,
(2) a soluble indicator reagent, comprising a second specific binding member conjugated to a label capable of producing a detectable signal, wherein said second specific binding member directly binds the analyte or indirectly binds the analyte by means of an ancillary specific binding member which is specific for the analyte, and
(3) an insoluble solid phase material comprising a second charged substance which is oppositely charged with respect to said first charged substance;
b) contacting the fluid sample with said capture reagent, whereby said capture reagent becomes bound to the analyte in the sample, thereby forming a capture reagent/analyte complex;
c) contacting said capture reagent/analyte complex with said indicator reagent, whereby said indicator reagent becomes bound to the analyte, thereby forming a capture reagent/analyte/indicator reagent sandwich complex;
d) separating said sandwich complex from the sample by contacting the sample with said solid phase material, whereby said solid phase material attracts and attaches to said first charged substance of said sandwich complex, thereby separating said sandwich complex from the sample; and
e) detecting said label associated with said solid phase or unbound indicator reagent as an indication of the presence or amount of the analyte in the sample.

4. The process according to Claim 3, wherein said capture reagent and said indicator reagent are simultaneously contacted with the fluid sample.

5. The process according to Claim 3, wherein at least one of said capture reagent and said indicator reagent indirectly binds to the analyte by means of an ancillary specific binding member specific for the analyte.

6. A process for detecting an analyte in a fluid sample, comprising:
a) providing
1) a soluble capture reagent comprising a first specific binding member conjugated to a first charged substance, wherein said first specific binding member directly binds the analyte or indirectly binds the analyte by means of an ancillary specific binding member which is specific for the analyte,
2) a soluble indicator reagent, comprising a second specific binding member conjugated to a label capable of producing a detectable signal, wherein said second specific binding member directly binds said first specific binding member or indirectly binds said first specific binding member by means of said ancillary specific binding member which is specific for said first specific binding member, and
3) an insoluble solid phase material comprising a second charged substance which is oppositely charged with respect to said first charged substance;
b) contacting the fluid sample with said capture reagent and said indicator reagent, whereby the analyte in the sample competes with said indicator reagent to bind to said capture reagent, thereby forming capture reagent/analyte complex and capture reagent/indicator reagent complex;
c) separating said complexes from the sample by contacting the sample with said solid phase material, whereby said solid phase material attracts and attaches to said first charged substance, thereby separating said complexes from the sample; and
d) detecting said label associated with said solid phase or unbound indicator reagent as an indication of the presence or amount of analyte in the sample.

7. The process according to Claim 6, wherein said capture reagent and said indicator reagent are sequentially contacted to the fluid sample.

8. The process according to Claim 6, wherein said capture reagent indirectly binds said indicator reagent or the analyte by means of an ancillary specific binding member.

9. The process according to Claims 1, 3 and 6, wherein said first charged substance is an anionic substance and said second charged substance is a cationic substance.

10. The process according to Claims 1, 3 and 6, wherein said insoluble solid phase material comprises a plurality of charged microparticles, said microparticles being retained by a solid phase base material, said microparticles being oppositely charged with respect to said first charged substance.

11. A sandwich immunoassay kit for determining the presence or amount of an analyte in a fluid sample, comprising:
a) a soluble capture reagent, comprising a first specific binding member conjugated to a first charged substance, wherein said first specific binding member directly binds the analyte or indirectly binds the analyte by means of an ancillary specific binding member which is specific for the analyte;
b) a soluble indicator reagent, comprising a second specific binding member conjugated to a label capable of producing a detectable signal, wherein said second specific binding member directly binds the analyte or indirectly binds the analyte by means of an ancillary specific binding member which is specific for the analyte; and
c) an insoluble solid phase material comprising a second charged substance which is oppositely charged with respect to said first charged substance.

12. A competitive immunoassay kit for determining the presence or amount of an analyte in a fluid sample, comprising:
a) a soluble capture reagent, comprising a first specific binding member conjugated to a first charged substance, wherein said first specific binding member directly binds the analyte or indirectly binds the analyte by means of an ancillary specific binding member which is specific for the analyte;
b) a soluble indicator reagent, comprising a second specific binding member conjugated to a label capable of producing a detectable signal, wherein said second specific binding member directly binds said first specific binding member so that it competes with said analyte for binding sites on said first specific binding member or indirectly binds said first specific binding member by means of said ancillary specific binding member which is specific for binding sites on said first specific binding member, said second specific binding member competing with the analyte for binding sites on said ancillary specific binding member; and
c) an insoluble solid phase material comprising a second charged substance which is oppositely charged with respect to said first charged substance.

13. A sandwich immunoassay kit for determining the presence or amount of an analyte in a fluid sample, comprising:
a) a soluble capture reagent, comprising a first specific binding member conjugated to a first charged substance, wherein said first specific binding member directly binds the analyte or indirectly binds the analyte by means of an ancillary specific binding member which is specific for the analyte;
b) a soluble indicator reagent, comprising a second specific binding member conjugated to a label capable of producing a detectable signal, wherein said second specific binding member directly binds the analyte or indirectly binds the analyte by means of an ancillary specific binding member which is specific for the analyte; and
c) an insoluble solid phase material comprising microparticles coated with a second charged substance which is oppositely charged with respect to said first charged substance, wherein said microparticles are retained on a solid phase base material.

14. A competitive immunoassay kit for determining the presence or amount of an analyte in a fluid sample, comprising:
a) a soluble capture reagent, comprising a first specific binding member conjugated to a first charged substance, wherein said first specific binding member directly binds the analyte or indirectly binds the analyte by means of an ancillary specific binding member which is specific for the analyte;
b) a soluble indicator reagent, comprising a second specific binding member conjugated to a label capable of producing a detectable signal, wherein said second specific binding member directly binds said first specific binding member so that it competes with said analyte for binding sites on said first specific binding member or indirectly binds said first specific binding member by means of said ancillary specific binding member which is specific for binding sites on said first specific binding member, said second specific binding member competing with the analyte for binding sites on said ancillary specific binding member; and
c) an insoluble solid phase material comprising microparticles coated with a second charged substance which is oppositely charged with respect to said first charged substance, wherein said microparticles are retained on a solid phase base material.

15. A complex comprising a soluble capture reagent comprising a specific binding member conjugated to a charged substance, and a solid phase containing a second charged substance which is oppositely charged with respect to said first charged substance, wherein said complex is formed by the attraction and attachment of said first and second substances.

16. A method for forming a complex comprising the step of contacting a solid phase with a fluid sample containing a soluble capture reagent comprising a specific binding member conjugated to a charged substance, said solid phase containing a second charged substance which is oppositely charged with respect to said first charged substance, wherein said complex is formed by the attraction and attachment of said first and second substances.

## Patentansprüche

1. Verfahren zum Abtrennen eines Analyten aus einer flüssigen Probe, das folgendes umfaßt:
a) das Bereitstellen
(1) eines löslichen Einfangreagenzes, das ein spezifisch bindendes Glied umfaßt, das an eine erste geladene Substanz konjugiert ist, wobei das spezifisch bindende Glied den Analyten direkt oder den Analyten indirekt mittels eines spezifisch bindenden Hilfsgliedes bindet, das für den Analyten spezifisch ist, und
(2) eines unlöslichen Festphasenmaterials, das eine zweite geladene Substanz umfaßt, die in Bezug auf die erste geladene Substanz entgegengesetzt geladen ist;
b) das In-Kontakt-Bringen der flüssigen Probe mit dem Einfangreagenz, wobei das Einfangreagenz an den Analyten in der Probe gebunden wird, wodurch sich ein Einfangreagenz/Analyt-Komplex ausbildet, und
c) das Abtrennen des Einfangreagenz/Analyt-Komplexes aus der Probe, indem die Probe mit dem Festphasenmaterial in Kontakt gebracht wird, wodurch das Festphasenmaterial die erste geladene Substanz anzieht und sich an diese anbindet, wodurch der Einfangreagenz/Analyt-Komplex aus der Probe abgetrennt wird.

2. Verfahren gemäß Anspruch 1, worin das Einfangreagenz den Analyten indirekt mittels eines spezifisch bindenden Hilfsgliedes bindet, das für den Analyten spezifisch ist.

3. Verfahren zum Nachweis eines Analyten in einer flüssigen Probe, das folgendes umfaßt:
a) das Bereitstellen
(1) eines löslichen Einfangreagenzes, das ein erstes spezifisch bindendes Glied umfaßt, das an eine erste geladene Substanz konjugiert ist, wobei das erste spezifisch bindende Glied den Analyten direkt oder den Analyten indirekt mittels eines spezifisch bindenden Hilfsgliedes bindet, das für den Analyten spezifisch ist,
(2) eines löslichen Indikatorreagenzes, das ein zweites spezifisch bindendes Glied umfaßt, das an eine Markierung konjugiert ist, die zur Erzeugnung eines nachweisbaren Signals in der Lage ist, wobei das zweite spezifisch bindende Glied den Analyten direkt oder den Analyten indirekt mittels eines spezifisch bindenden Hilfsgliedes bindet, das für den Analyten spezifisch ist, und
(3) eines unlöslichen Festphasenmaterials, das eine zweite geladene Substanz umfaßt, die in Bezug auf die erste geladene Substanz entgegengesetzt geladen ist;
b) das In-Kontakt-Bringen der flüssigen Probe mit dem Einfangreagenz, wodurch das Einfangreagenz an den Analyten in der Probe gebunden wird, wobei sich ein Einfangreagenz/Analyt-Komplex ausbildet;
c) das In-Kontakt-Bringen des Einfangreagenz/Analyt-Komplexes mit dem Indikatorreagenz, wobei das Indikatorreagenz an den Analyten gebunden wird, wobei sich ein Einfangreagenz/Analyt/Indikatorreagenz-Sandwichkomplex ausbildet;
d) das Abtrennen des Sandwichkomplexes aus der Probe, indem die Probe mit dem Festphasenmaterial in Kontakt gebracht wird, wobei die feste Phase die erste geladene Substanz des Sandwichkomplexes anzieht und sich an diese anbindet, wobei der Sandwichkomplex aus der Probe abgetrennt wird; und
e) das Nachweisen der Markierung, die mit der festen Phase verbunden ist, oder aber ungebundenen Indikatorreagenzes als Anzeige für die Anwesenheit oder Menge des Analyten in der Probe.

4. Verfahren gemäß Anspruch 3, wobei das Einfangreagenz und das Indikatorreagenz gleichzeitig mit der flüssigen Probe in Kontakt gebracht werden.

5. Verfahren nach Anspruch 3, wobei wenigstens das Einfangreagenz oder das Indikatorreagenz indirekt an den Analyten mittels eines spezifisch bindenden Hilfsgliedes bindet, das für den Analyten spezifisch ist.

6. Verfahren zum Nachweis eines Analyten in einer flüssigen Probe, das folgendes umfaßt:
a) das Bereitstellen
1) eines löslichen Einfangreagenzes, das ein erstes spezifisch bindendes Glied umfaßt, das an eine erste geladene Substanz konjugiert ist, wobei das erste spezifisch bindende Glied den Analyten direkt oder den Analyten indirekt mittels eines spezifisch bindenden Hilfsgliedes bindet, das für den Analyten spezifisch ist;
2) eines löslichen Indikatorreagenzes, das ein zweites spezifisch bindendes Glied umfaßt, das an eine Markierung konjugiert ist, die zur Erzeugung eines nachweisbaren Signals befähigt ist, wobei das zweite spezifisch bindende Glied das erste spezifisch bindende Glied direkt oder das erste spezifisch bindende Glied indirekt mittels eines spezifisch bindenden Hilfsgliedes bindet, das für das erste spezifisch bindende Glied spezifisch ist, und
3) eines unlöslichen Festphasenmaterials, das eine zweite geladene Substanz umfaßt, die in Bezug auf die erste geladene Substanz entgegengesetzt geladen ist;
b) das In-Kontakt-Bringen der flüssigen Probe mit dem Einfangreagenz und dem Indikatorreagenz, wobei der Analyt in der Probe mit dem Indikatorreagenz um die Bindung an das Einfangreagenz konkurriert, wobei sich ein Einfangreagenz/Analyt-Komplex und ein Einfangreagenz/Indikatorreagenz-Komplex ausbilden;
c) das Abtrennen der Komplexe aus der Probe durch In-Kontakt-Bringen der Probe mit dem Festphasenmaterial, wobei das Festphasenmaterial die erste geladene Substanz anzieht und sich an diese anbindet, wobei die Komplexe aus der Probe abgetrennt werden; und
d) das Nachweisen der Markierung, die mit der festen Phase verbunden ist oder des ungebundenen Indikatorreagenzes als Anzeige für die Anwesenheit oder Menge an Analyt in der Probe.

7. Verfahren nach Anspruch 6, wobei das Einfangreagenz, und das Indikatorreagenz nacheinander mit der flüssigen Probe in Kontakt gebracht werden.

8. Verfahren nach Anspruch 6, wobei das Einfangreagenz das Indikatorreagenz oder den Analyten indirekt mittels eines spezifisch bindenden Hilfsgliedes bindet.

9. Verfahren nach einem der Ansprüche 1, 3 und 6, wobei die erste geladene Substanz eine anionische Substanz ist, und wobei die zweite geladene Substanz eine kationische Substanz ist.

10. Verfahren nach einem der Ansprüche 1, 3 und 6, wobei das unlösliche Festphasenmaterial eine Vielzahl von geladenen Mikropartikeln umfaßt, wobei die Mikropartikel von einem Festphasengrundmaterial zurückgehalten werden, wobei die Mikropartikel in Bezug auf die erste geladene Substanz entgegengesetzt geladen sind.

11. Ein Sandwichimmunoassaykit zur Bestimmung der Anwesenheit oder Menge eines Analyten in einer flüssigen Probe, das folgendes umfaßt:
a) ein lösliches Einfangreagenz, das ein erstes spezifisch bindendes Glied umfaßt, das an eine erste geladene Substanz konjugiert ist, wobei das erste spezifisch bindende Glied den Analyten direkt oder den Analyten indirekt mittels eines spezifisch bindenden Hilfsgliedes bindet, das für den Analyten spezifisch ist;
b) ein lösliches Indikatorreagenz, das ein zweites spezifisch bindendes Glied umfaßt, das an einer Markierung konjugiert ist, die zur Erzeugung eines nachweisbaren Signals befähigt ist, wobei das zweite spezifisch bindende Glied den Analyten direkt oder den Analyten indirekt mittels eines spezifisch bindenden Hilfsgliedes bindet, das für den Analyten spezifisch ist; und c) ein unlösliches Festphasenmaterial, das eine zweite geladene Substanz umfaßt, die im Bezug auf die erste geladene Substanz entgegengesetzt geladen ist.

12. Konkurrenzimmunoassaykit zur Bestimmung der Anwesenheit oder Menge eines Analyten in einer flüssigen Probe, das folgendes umfaßt:
a) ein lösliches Einfangreagenz, das ein erstes spezifisch bindendes Glied umfaßt, das an eine erste geladene Substanz konjugiert ist, wobei das erste spezifisch bindende Glied den Analyten direkt bindet oder den Analyten indirekt mittels eines spezifisch bindenden Hilfsgliedes bindet, das für den Analyten spezifisch ist;
b) ein unlösliches Indikatorreagenz, das ein zweites spezifisch bindendes Glied umfaßt, das an eine Markierung konjugiert ist, die zur Erzeugung eines nachweisbaren Signals fähig ist, wobei das zweite spezifisch bindende Hilfsglied das erste spezifisch bindende Glied direkt bindet, so daß es mit dem Analyten um die Bindungszentren auf dem ersten spezifisch bindenden Glied konkurriert oder wobei es das erste spezifisch bindende Glied indirekt mittels des spezifisch bindenden Hilfsgliedes bindet, das für die Bindungszentren auf dem ersten spezifisch bindenden Glied spezifisch ist, wobei das zweite spezifisch bindende Glied mit dem Analyten um die Bindungszentren auf dem spezifisch bindenden Hilfsglied konkurriert; und
c) ein unlösliches Festphasenmaterial, das eine zweite geladene Substanz umfaßt, die in Bezug auf die erste geladene Substanz entgegengesetzt geladen ist.

13. Sandwichimmunoassaykit zur Bestimmung der Anwesenheit oder Menge eines Analyten in einer flüssigen Probe, das folgendes umfaßt:
a) ein lösliches Einfangreagenz, das ein erstes spezifisch bindendes Glied umfaßt, das an eine erste geladene Substanz konjugiert ist, wobei das erste spezifisch bindende Glied den Analyten direkt bindet oder den Analyten indirekt mittels eines spezifisch bindenden Hilfsgliedes bindet, das für den Analyten spezifisch ist;
b) ein lösliches Indikatorreagenz, das ein zweites spezifisch bindendes Glied umfaßt, das an eine Markierung konjugiert ist, die zur Erzeugung eines nachweisbaren Signals fähig ist, wobei das zweite spezifisch bindende Glied den Analyten direkt bindet oder den Analyten indirekt mittels eines spezifisch bindenden Hilfsgliedes bindet, das für den Analyten spezifisch ist; und c) ein unlöslichen Festphasenmaterial, das Mikropartikel umfaßt, die mit einer zweiten geladenen Substanz beschichtet sind, die in Bezug auf die erste geladene Substanz entgegengesetzt geladen ist, wobei die Mikropartikel auf einem Festphasengrundmaterial zurückgehalten werden.

14. Konkurrenzimmunoassaykit zur Bestimmung der Anwesenheit oder Menge eines Analyten in einer flüssigen Probe, das folgendes umfaßt:
a) ein lösliches Einfangreagenz, das ein erstes spezifisch bindendes Glied umfaßt, das an eine erste geladene Substanz konjugiert ist, wobei das erste spezifisch bindende Glied den Analyten direkt oder den Analyten indirekt mittels eines spezifisch bindenden Hilfsgliedes bindet, das für den Analyten spezifisch ist;
b) ein lösliches Indikatorreagenz, das ein zweites spezifisch bindendes Glied umfaßt, das an eine Markierung konjugiert ist, die zur Erzeugung eines nachweisbaren Signals fähig ist, wobei das zweite spezifisch bindende Glied das erste spezifisch bindende Glied direkt bindet, sc daß es mit dem Analyten um die Bindungszentren auf dem ersten spezifisch bindenden Glied konkurriert oder wobei es das erste spezifisch bindende Glied indirekt mittels des spezifisch bindenden Hilfsgliedes bindet, das für die Bindungszentren auf dem ersten spezifischen Bindungsglied spezifisch ist, wobei das zweite spezifisch bindende Glied mit dem Analyten um die Bindungszentren auf dem spezifisch bindenden Hilfsglied konkurriert; und
c) ein unlösliches Festphasenmaterial, das Mikropartikel umfaßt, die mit einer zweiten geladenen Substanz beschichtet sind, die in Bezug auf die erste geladene Substanz entgegengesetzt geladen ist, wobei die Mikropartikel auf einem Festphasengrundmaterial zurückgehalten werden.

15. Komplex, der ein lösliches Einfangreagenz umfaßt, welches ein spezifisch bindendes Glied umfaßt, das an eine geladene Substanz konjugiert ist, und eine feste Phase, die eine zweite geladene Substanz enthält, die in Bezug auf die erste geladene Substanz entgegengesetzt geladen ist, wobei der Komplex durch die Anziehung und die Anbindung der ersten und der zweiten Substanz ausgebildet wird.

16. Verfahren zur Ausbildung eines Komplexes, das den Schritt des In-Kontakt-Bringens einer festen Phase mit einer flüssigen Probe umfaßt, die ein lösliches Einfangreagenz enthält, das ein spezifisch bindendes Glied umfaßt, das an eine geladene Substanz konjugiert ist, wobei die feste Phase eine zweite geladene Substanz enthält, die in Bezug auf die erste geladene Substanz entgegengesetzt geladen ist, wobei der Komplex durch die Anziehung und die Anbindung der ersten und der zweiten Substanz ausgebildet wird.

## Revendications

1. Procédé pour la séparation d'un analyte d'un échantillon fluide, comprenant
a) la fourniture
(1) d'un réactif de capture soluble comprenant un élément de fixation spécifique conjugué à une première substance chargée, ledit élément de fixation spécifique se liant directement à l'analyte ou se liant indirectement à l'analyte au moyen d'un élément de fixation spécifique auxiliaire qui est spécifique de l'analyte, et
(2) d'une matière en phase solide insoluble comprenant une seconde substance chargée, qui a une charge opposée à celle de ladite première substance chargée ;
b) la mise en contact de l'échantillon fluide avec ledit réactif de capture, ledit réactif de capture se liant à l'analyte dans l'échantillon et formant ainsi un complexe réactif de capture/analyte ; et
c) la séparation dudit complexe réactif de capture/analyte de l'échantillon par la mise en contact de l'échantillon avec ladite matière en phase solide, ladite matière en phase solide attirant ladite première substance chargée et se fixant à elle, avec ainsi séparation dudit complexe réactif de capture/analyte de l'échantillon.

2. Procédé selon la revendication 1, dans lequel ledit réactif de capture se lie indirectement à l'analyte au moyen d'un élément de fixation spécifique auxiliaire spécifique de l'analyte.

3. Procédé de détection d'un analyte dans un échantillon fluide, comprenant
a) la fourniture
(1) d'un réactif de capture soluble, comprenant un premier élément de fixation spécifique conjugué à une première substance chargée, ledit premier élément de fixation spécifique se liant directement à l'analyte ou se liant indirectement à l'analyte au moyen d'un élément de fixation spécifique auxiliaire qui est spécifique de l'analyte,
(2) d'un réactif indicateur soluble, comprenant un second élément de fixation spécifique conjugué à un marqueur capable de produire un signal détectable, ledit second élément de fixation spécifique se liant directement à l'analyte ou se liant indirectement à l'analyte au moyen d'un élément de fixation spécifique auxiliaire qui est spécifique de l'analyte, et
(3) d'une matière en phase solide insoluble comprenant une seconde substance chargée, qui a une charge opposée à celle de ladite première substance chargée ;
b) la mise en contact de l'échantillon fluide avec ledit réactif de capture, ledit réactif de capture se liant à l'analyte dans l'échantillon et formant ainsi un complexe réactif de capture/analyte ;
c) la mise en contact dudit complexe réactif de capture/analyte avec ledit réactif indicateur, ledit réactif indicateur se liant à l'analyte et formant ainsi un complexe sandwich réactif de capture/analyte/réactif indicateur ;
d) la séparation dudit complexe sandwich de l'échantillon par la mise en contact de l'échantillon avec ladite matière en phase solide, ladite matière en phase solide attirant ladite première substance chargée dudit complexe sandwich et se fixant à elle, avec ainsi séparation dudit complexe sandwich de l'échantillon ; et
e) la détection dudit marqueur associé à ladite phase solide ou au dit réactif indicateur non lié comme indication de la présence ou de la quantité de l'analyte dans l'échantillon.

4. Procédé selon la revendication 3, dans lequel ledit réactif de capture et ledit réactif indicateur sont mis en contact avec l'échantillon fluide en même temps.

5. Procédé selon la revendication 3, dans lequel au moins un desdits réactif de capture et réactif indicateur se lie indirectement à l'analyte par l'intermédiaire d'un élément de fixation spécifique auxiliaire spécifique de l'analyte.

6. Procédé de détection d'un analyte dans un échantillon fluide, comprenant
a) la fourniture
(1) d'un réactif de capture soluble, comprenant un premier élément de fixation spécifique conjugué à une première substance chargée, ledit premier élément de fixation spécifique se liant directement à l'analyte ou se liant indirectement à l'analyte au moyen d'un élément de fixation spécifique auxiliaire qui est spécifique de l'analyte,
(2) d'un réactif indicateur soluble, comprenant un second élément de fixation spécifique conjugué à un marqueur capable de produire un signal détectable, ledit second élément de fixation spécifique se liant directement au dit premier élément de fixation spécifique ou se liant indirectement au dit premier élément de fixation spécifique au moyen dudit élément de fixation spécifique auxiliaire qui est spécifique dudit premier élément de fixation spécifique, et
(3) d'une matière en phase solide insoluble comprenant une seconde substance chargée, qui a une charge opposée à celle de ladite première substance chargée ;
b) la mise en contact de l'échantillon fluide avec ledit réactif de capture et ledit réactif indicateur, l'analyte dans l'échantillon entrant en compétition avec ledit réactif indicateur pour fixer ledit réactif de capture, avec ainsi formation d'un complexe réactif de capture/analyte et d'un complexe réactif de capture/réactif indicateur ;
c) la séparation desdits complexes de l'échantillon par la mise en contact de l'échantillon avec ladite matière en phase solide, ladite matière en phase solide attirant ladite première substance chargée et se fixant à elle, avec ainsi séparation desdits complexes de l'échantillon ; et
e) la détection dudit marqueur associé à ladite phase solide ou au dit réactif indicateur non lié comme indication de la présence ou de la quantité de l'analyte dans l'échantillon.

7. Procédé selon la revendication 6, dans lequel ledit réactif de capture et ledit réactif indicateur sont mis en contact avec l'échantillon fluide de façon séquentielle.

8. Procédé selon la revendication 6, dans lequel ledit réactif de capture se fixe indirectement au dit réactif indicateur ou à l'analyte au moyen d'un élément de fixation spécifique auxiliaire.

9. Procédé selon les revendications 1, 3 et 6, dans lequel ladite première substance chargée est une substance anionique, et ladite seconde substance chargée est une substance cationique.

10. Procédé selon les revendications 1, 3 et 6, dans lequel ladite matière insoluble en phase solide contient plusieurs microparticules chargées, lesdites microparticules étant retenues par une matière de base en phase solide, lesdites microparticules étant d'une charge opposée à celle de ladite première substance chargée.

11. Trousse de dosage immunologique par une méthode sandwich pour la détermination de la présence ou de la quantité d'un analyte dans un échantillon fluide, comprenant :
a) un réactif de capture soluble, comprenant un premier élément de fixation spécifique conjugué à une première substance chargée, ledit premier élément de fixation spécifique se liant directement à l'analyte ou se liant indirectement à l'analyte au moyen d'un élément de fixation spécifique auxiliaire qui est spécifique de l'analyte,
b) un réactif indicateur soluble, comprenant un second élément de fixation spécifique conjugué à un marqueur capable de produire un signal détectable, ledit second élément de fixation spécifique se liant directement à l'analyte ou se liant indirectement à l'analyte au moyen d'un élément de fixation spécifique auxiliaire qui est spécifique de l'analyte, et
c) une matière en phase solide insoluble comprenant une seconde substance chargée, qui a une charge opposée à celle de ladite première substance chargée.

12. Trousse de dosage immunologique compétitif pour la détermination de la présence ou de la quantité d'un analyte dans un échantillon fluide, comprenant :
a) un réactif de capture soluble, comprenant un premier élément de fixation spécifique conjugué à une première substance chargée, ledit premier élément de fixation spécifique se liant directement à l'analyte ou se liant indirectement à l'analyte au moyen d'un élément de fixation spécifique auxiliaire qui est spécifique de l'analyte,
b) un réactif indicateur soluble, comprenant un second élément de fixation spécifique conjugué à un marqueur capable de produire un signal détectable, ledit second élément de fixation spécifique se liant directement au dit premier élément de fixation spécifique si bien qu'il entre en compétition avec ledit analyte pour les sites de fixation sur ledit premier élément de fixation spécifique ou se liant indirectement au dit premier élément de fixation spécifique au moyen du dit élément de fixation spécifique auxiliaire qui est spécifique des sites de liaison sur ledit premier élément de fixation spécifique, ledit second élément de fixation spécifique entrant en compétition avec l'analyte pour les sites de liaison sur ledit élément de fixation spécifique auxiliaire ; et
c) une matière en phase solide insoluble comprenant une seconde substance chargée, qui a une charge opposée à celle de ladite première substance chargée.

13. Trousse de dosage immunologique par une méthode sandwich pour la détermination de la présence ou de la quantité d'un analyte dans un échantillon fluide, comprenant :
a) un réactif de capture soluble, comprenant un premier élément de fixation spécifique conjugué à une première substance chargée, ledit premier élément de fixation spécifique se liant directement à l'analyte ou se liant indirectement à l'analyte au moyen d'un élément de fixation spécifique auxiliaire qui est spécifique de l'analyte,
b) un réactif indicateur soluble, comprenant un second élément de fixation spécifique conjugué à un marqueur capable de produire un signal détectable, ledit second élément de fixation spécifique se liant directement à l'analyte ou se liant indirectement à l'analyte au moyen d'un élément de fixation spécifique auxiliaire qui est spécifique de l'analyte, et
c) une matière en phase solide insoluble comprenant des microparticules revêtues d'une seconde substance chargée, qui a une charge opposée à celle de ladite première substance chargée, lesdites microparticules étant retenues sur une matière de base en phase solide.

14. Trousse de dosage immunologique compétitif pour la détermination de la présence ou de la quantité d'un analyte dans un échantillon fluide, comprenant :
a) un réactif de capture soluble, comprenant un premier élément de fixation spécifique conjugué à une première substance chargée, ledit premier élément de fixation spécifique se liant directement à l'analyte ou se liant indirectement à l'analyte au moyen d'un élément de fixation spécifique auxiliaire qui est spécifique de l'analyte,
b) un réactif indicateur soluble, comprenant un second élément de fixation spécifique conjugué à un marqueur capable de produire un signal détectable, ledit second élément de fixation spécifique se liant directement au dit premier élément de fixation spécifique si bien qu'il entre en compétition avec ledit analyte pour les sites de fixation sur ledit premier élément de fixation spécifique ou se liant indirectement au dit premier élément de fixation spécifique au moyen dudit élément de fixation spécifique auxiliaire qui est spécifique des sites de liaison sur ledit premier élément de fixation spécifique, ledit second élément de fixation spécifique entrant en compétition avec l'analyte pour les sites de liaison sur ledit élément de fixation spécifique auxiliaire ; et
c) une matière en phase solide insoluble comprenant des microparticules revêtues d'une seconde substance chargée, qui a une charge opposée à celle de ladite première substance chargée, lesdites microparticules étant retenues sur une matière de base en phase solide.

15. Complexe comprenant un réactif de capture soluble comprenant un élément de fixation spécifique conjugué à une substance chargée, et une phase solide contenant une seconde substance chargée qui a une charge opposée à celle de ladite première substance chargée, ledit complexe étant formé par l'attraction entre la première et la seconde substance et leur liaison.

16. Méthode pour la formation d'un complexe comprenant l'étape de mise en contact d'une phase solide avec un échantillon fluide contenant un réactif de capture soluble comprenant un élément de fixation spécifique conjugué à une substance chargée, ladite phase solide contenant une seconde substance chargée qui a une charge opposée à celle de ladite première substance chargée, ledit complexe étant formé par l'attraction entre la première et la seconde substance et leur liaison.
